# EUROPEAN PATENT APPLICATION

(11) **EP 4 497 745 A1**
(43) Date of publication of application: **29.01.2025**
(21) Application number: 22932607.9
(22) Date of filing: 23.03.2022
(51) Int. Cl.: C07D 207/27, C07B 41/08, C07B 41/12, C07B 63/02

(54) **METHOD FOR PURIFYING LEVETIRACETAM INTERMEDIATE**

(71) Applicant: Zhejiang Huahai Pharmaceutical Co., Ltd., Taizhou, Zhejiang 317024 (CN)
(72) Inventor: YAN, Fengfeng, Zhejiang 317024 (CN); CHEN, Hui, Zhejiang 317024 (CN); GONG, Yulong, Zhejiang 317024 (CN); ZHU, Yuanxun, Zhejiang 317024 (CN)
(74) Representative: Schmitz, Joseph
(86) International application number: PCT/CN2022/082401
(87) International publication number: WO 2023/178538

(57) **Abstract**

The present invention relates to the field of pharmaceutical chemical engineering, in particular to a method for purifying a levetiracetam intermediate, which comprises the following steps: (1) hydrolyzing and acidifying a crude product of a compound of formula III and performing crystallization to obtain a compound of formula IV; and (2) subjecting the compound of formula IV to an esterification reaction and separation to obtain a purified compound of formula III. The present method has a purification effect obviously superior to that of a physical purification mode, can reach a total yield of 90% or above, and has a great practical value.

## Description

### TECHNICAL FIELD

The present invention relates to the field of pharmaceutical chemical engineering, in particular to a method for purifying a levetiracetam intermediate.

### BACKGROUND

Levetiracetam (trade name: Keppra), a new antiepileptic drug developed by UCB, S.A., BE, is an acetylpyrrolidine compound, chemically named as (S)-α-ethyl-2-oxo-1-pyrrolidine acetamide, with a structure shown in formula I:

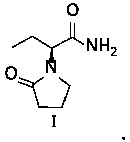

UCB, S.A. has reported a method for obtaining levetiracetam by ammonolysis of (S)-α-ethyl-2-oxo-1-pyrrolidine acetate (II) in the presence of aqueous ammonia in the patent US7,122,682, and the synthesis route is as follows:

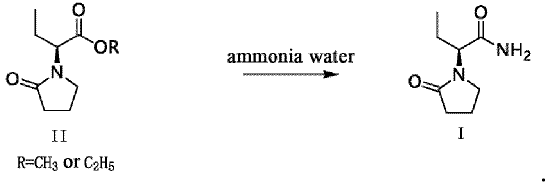

The compound of formula II can be obtained by condensation and resolution of 2-halobutyrate and 2-pyrrolidone in the presence of a strong alkali, and the synthesis route is as follows:

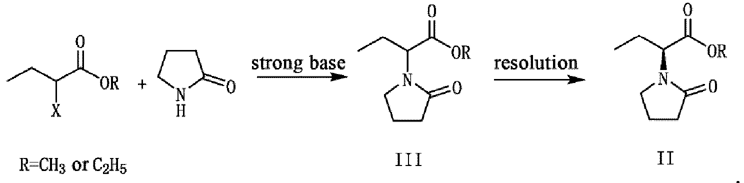

After the condensation reaction is completed, potable water is added to quench the reaction, then the reaction solution is extracted with an organic solvent, and the organic layers are combined and concentrated to obtain the compound of formula III. The compound of formula III is an oily liquid, which cannot be purified by conventional crystallization. Generally, it is directly used in the next step of resolution reaction without purification, and the compound of formula II is obtained after resolution. The resolution process has no purification effect, so the resolution will only affect the purity of isomers, and other impurities are the same as those in compound of formula III, that is, the impurities in compound of formula III will enter into compound of formula II without any reservation. However, these impurities are not treated in this route, and the impurities in the condensate will be ammoniated with ammonia in the crude product stage to form corresponding derivative impurities, which will affect the quality of subsequent crude and finished levetiracetam as a drug.

In order to solve the above impurity problem, a reaction purification method is developed to purify compound of formula III, which can greatly improve the purity of compound of formula III and ensure the quality and safety of subsequent compound of formula III and levetiracetam drugs.

### SUMMARY

The purpose of the present invention is to provide a method for purifying a levetiracetam intermediate, which can greatly improve the purity of compound of formula III and ensure the quality and safety of subsequent compound of formula III and levetiracetam drugs.

In order to achieve the above object, the present invention adopts the following technical solution: a method for purifying a levetiracetam intermediate, comprising:
(1) hydrolyzing a crude product of a compound of formula III under alkaline conditions, followed by acidifying, then crystallizing to obtain a compound of formula IV; and
(2) subjecting the compound of formula IV to an esterification reaction, followed by separation, to obtain a purified compound of formula III;
   wherein the compound of formula III is 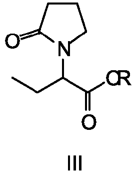 , R is methyl or ethyl, and the compound of formula IV is

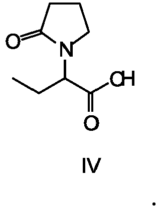

In some embodiments, the step (1) comprises the following steps:
(a) mixing the crude product of the compound of formula III with an aqueous solution of inorganic alkali, and then heating and performing reaction;
(b) adjusting pH to a pH number between 0.5 and 3.5; and
(c) lowering temperature, and crystallizing, filtering and drying a mixture obtained in step (b) to obtain the compound of formula IV.

In some embodiments, in the step (a), a temperature of the reaction is 35°C to 65°C, and reaction time is 1 to 5 hours.
and/or
In some embodiments, in the step (a), the inorganic alkali is one selected from the group consisting of sodium hydroxide and potassium hydroxide, a concentration of the aqueous solution of inorganic alkali is 5wt% to 30wt%, and a weight of the aqueous solution of inorganic alkali is 1 to 10 times a weight of the compound of formula III.

In some embodiments, in the step (a),
the temperature of the reaction is 45°C to 55°C, such as 45°C, 50°C or 55°C; the inorganic alkali is sodium hydroxide.

In some embodiments, in the step (b), the pH is adjusted to a pH number between 2.0 and 2.5.

In some embodiments, in the step (c), a temperature after lowering temperature is -10°C to 10°C, a duration of the crystallization is 1 hour to 2 hours, a temperature for the drying is 80°C to 100°C, and an end point of the drying is until a moisture content :::;0.5wt%.

In some embodiments, the step (2) comprises the following steps:
(d) adding an alcohol solvent into the compound of formula IV, adding an esterification reaction catalyst, and then heating and performing reaction;
(e) adding water, adjusting the pH to a pH number between 6 and 8 with alkali, and distilling; and
(f) extracting with an organic solvent, and distilling an obtained extract to obtain the purified compound of formula III.

In some embodiments, in the step (d),
the alcohol solvent is selected from the group consisting of methanol, ethanol, and a mixture thereof, and a weight of the alcohol solvent is 0.5 to 10 times a weight of the compound of formula III; and/or
the esterification reaction catalyst is 90wt% to 98wt% concentrated sulfuric acid, and a weight of the concentrated sulfuric acid is 0.01 to 0.2 times the weight of the compound of formula III; and/or
a temperature of the reaction is 50°C to 75°C and reaction time is 1 hour to 5 hours.

In some embodiments, in the step (e),
a weight of added water is 0.5 to 5 times the weight of the compound of formula III.

In some embodiments, in the step (e), the alkali is one or more selected from the group consisting of sodium hydroxide, potassium hydroxide, sodium carbonate, potassium carbonate, sodium bicarbonate and potassium bicarbonate.

In some embodiments, in the step (e), a weight of distilled solvent is 0.8 to 1.2 times a weight of added alcohol solvent, and a temperature of the distilling is ≤70°C.

In some embodiments, in the step (e),
a weight of added water is 0.5 to 5 times a weight of the compound of formula III; the alkali is one or more selected from the group consisting of sodium hydroxide, potassium hydroxide, sodium carbonate, potassium carbonate, sodium bicarbonate and potassium bicarbonate; and a weight of distilled solvent is 0.8 to 1.2 times a weight of added alcohol solvent, and a temperature of the distilling is ≤70°C.

In some embodiments, in the step (f), the organic solvent is one or more selected from the group consisting of toluene, benzene, dichloromethane and ethyl acetate, the extraction is performed 2 to 5 times, a weight of the organic solvent for a single extraction is 1 to 10 times a weight of the compound of formula III, and a temperature of the distilling is ≤70°C.

In some embodiments, in the step (e), the alkali is sodium carbonate.

In some embodiments, in the step (f), the organic solvent is dichloromethane.

The method comprises the following steps: firstly, hydrolyzing compound of formula III with the solution of alkali to obtain compound of formula IV, and then subjecting compound of formula IV to an esterification reaction to obtain purified compound of formula III. The compound of formula III is purified by reaction purification, and the purification effect is obviously superior to that of a physical purification, with a total yield of up to 90% or more, thus having a great practical value.

### DETAILED DESCRIPTION

### Example 1:

### Preparation of methyl α-ethyl-2-oxo-1-pyrrolidine acetate

2300g of toluene was taken and slowly added with 240g of sodium hydride while controlling the temperature at 0°C, followed by slowly adding 1000g of 2-pyrrolidone dropwise. The temperature was raised to 55°C, and 2000g of methyl 2-bromobutyrate was slowly added dropwise. The temperature was maintained for 6 hours, and 2000g of potable water was added. The phases were separated, the water phase was extracted twice with 2000g of toluene, and the toluene phases were combined. The combined toluene phase was concentrated under reduced pressure at a temperature <70°C until no solvent residue, and 1941g of methyl α-ethyl-2-oxo-1-pyrrolidine acetate with a purity of 94.7% was obtained.

### Purification of methyl α-ethyl-2-oxo-1-pyrrolidine acetate

100g of methyl α-ethyl-2-oxo-1-pyrrolidine acetate (purity: 94.7%) was added into a reactor, followed by adding 200g of 15wt% sodium hydroxide aqueous solution. The temperature was raised to 45°C, and the reaction was carried out at a maintained temperature of 45°C for 2 hours. After confirming there was no raw material residue by TLC (developing agent: acetone:ethyl acetate=2:3, iodine fumigation color development, the retardation factor value of raw materials: 0.8), the pH of the reaction solution was adjusted to 2.0 with the addition of 36wt% refined hydrochloric acid. When materials were precipitated from the reaction solution along with the addition of hydrochloric acid, the pH adjustment was stopped. The materials were slowly cooled down to 0°C, stirred at a maintained temperature of 0°C for 2 hours, and filtered. The filter cake was dried at 95°C for 5 hours, the moisture content of which was detected to be 0.1wt%, and 90.6g (98%) of compound of formula IV was obtained.

The whole batch of compound of formula IV was added into a reactor, followed by adding 100g of methanol. The temperature was 0°C, and 4g of 98wt% concentrated sulfuric acid was slowly added dropwise. The temperature was raised to 65°C upon the completion of dropwise addition, and the reaction was carried out for 2 hours. After confirming there was no raw material residue by TLC (developing agent: acetone:ethyl acetate=2:3, iodine fumigation color development, the retardation factor value of raw materials: 0.15), 100g of potable water was added, and the pH was adjusted to a pH number between 7 and 8 with the slow addition of sodium carbonate. The mixture was distilled under reduced pressure at a temperature <50°C, and when 100g of solvent was obtained by the distilling, the distilling was stopped. The resultant was extracted with 150g of dichloromethane added, and the water phase was extracted with 150g of dichloromethane for another three times. The dichloromethane phases were combined, and the combined phase was distilled under reduced pressure at a temperature <50°C until no solvent residue to obtain 93g of methyl α-ethyl-2-oxo-1-pyrrolidine acetate. The total yield of the two-step reaction was 93% and the purity was 99.9%.

According to the research of the inventors, if the synthesis route of patent US7122682 mentioned in the background is used and the compound of formula III obtained after condensation is not treated, the impurities in the condensate mainly include the following:

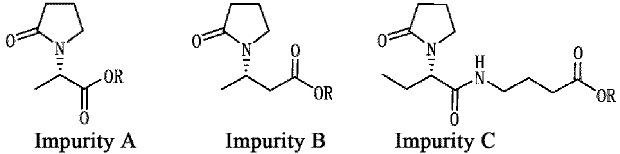

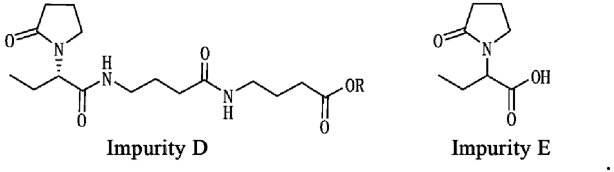

The inventors tried to purify compound of formula III by acid washing, water washing and alkali washing, and the results showed that the impurity removal effects of all the methods were not ideal. After purified by the method of the present invention, the contents of the above impurities were significantly reduced, and the quality comparison before and after purification is shown in the table as below.

| sample | purity | Impurity A | Impurity B | Impurity C | Impurity D | Impurity E | Maximum amount of other impurities |
|---|---|---|---|---|---|---|---|
| Before purification | 94.7% | 0.25% | 0.31% | 2.25% | 0.23% | 1.70% | 0.21% |
| After purification | 99.9% | 0.003% | N.D | N.D | N.D | 0.005% | N.D |

### Comparative Example

100g of methyl α-ethyl-2-oxo-1-pyrrolidine acetate in Example 1 was taken as the raw material and washed with potable water, dilute acid and dilute alkali, respectively. The amount of each washing agent was 150g, and the washing duration was 0.5 hour. The data before and after washing is as follows.

| sample | purity | Impurity A | Impurity B | Impurity C | Impurity D | Impurity E | Maximum amount of other impurities |
|---|---|---|---|---|---|---|---|
| Before purification | 93.50% | 0.23% | 0.41% | 2.71% | 0.12% | 2.51% | 0.18% |
| Washed with potable water | 94.10% | 0.23% | 0.35% | 2.65% | 0.08% | 1.80% | 0.15% |
| 10% Sodium carbonate solution | 96.20% | 0.20% | 0.12% | 1.85% | 0.11% | 0.32% | 0.17% |
| 5% Sodium hydroxide solution | 94.90% | 0.21% | 0.23% | 1.75% | 0.10% | 1.79% | 0.20% |
| 0.5 mol/L hydrochloric acid | 93.10% | 0.24% | 0.08% | 2.55% | 0.06% | 2.78% | 0.17% |
| 0.2 mol/L Sulfuric acid solution | 93.23% | 0.22% | 0.05% | 2.61% | 0.03% | 2.81% | 0.18% |

From the Comparative Example, it can be seen that the effects of purifying the crude intermediate using methods such as acid washing, water washing, alkali washing are poor, while the purity of methyl α-ethyl-2-oxo-1-pyrrolidine acetate prepared by the purification in Example 1 of the present invention could reach up to 99.9%. The contents of related impurities were also significantly reduced, and impurity E with the maximum content was only 0.005%. The present method has a purification effect obviously superior to that of a physical purification, and the purification method of the present invention can achieve a total yield of up to 90% or more, thus having a great practical value.

### Example 2:

### Preparation of ethyl α-ethyl-2-oxo-1-pyrrolidine acetate

1515g of ethyl α-ethyl-2-oxo-1-pyrrolidine acetate was prepared according to the process of Example 1, and the purity was 93.5%.

### Purification of ethyl α-ethyl-2-oxo-1-pyrrolidine acetate

100g of ethyl α-ethyl-2-oxo-1-pyrrolidine acetate (purity: 93.5%) was added into a reactor, followed by the addition of 120g of 30wt% sodium hydroxide aqueous solution. The temperature was raised to 55°C, and the reaction was carried out at a maintained temperature of 55°C for 2.5 hours. After confirming there was no raw material residue by TLC (developing agent: acetone: ethyl acetate=2:3, iodine fumigation color development, the retardation factor value of raw materials: 0.8), the pH of the reaction solution was adjusted to 2.5 with the addition of 36wt% refined hydrochloric acid. When materials were precipitated from the reaction solution along with the addition of hydrochloric acid, the pH adjustment was stopped. The materials were slowly cooled down to 0°C, stirred at a maintained temperature of 0°C for 1.5 hours, and filtered. The filter cake was dried at 95°C for 5 hours, the moisture content of which was detected to be 0.2wt%, and 83.4g(97%) of compound of formula IV was obtained.

The whole batch of compound of formula IV was added into a reactor, followed by the addition of 200g of ethanol. The temperature was 0°C, and 6g of 98 wt% concentrated sulfuric acid was slowly added dropwise. The temperature was raised to 75°C upon the completion of dropwise addition, and the reaction was carried out for 4 hours. After confirming there was no raw material residue by TLC (developing agent: acetone:ethyl acetate=2:3, and iodine fumigation color development, the retardation factor value of raw materials: 0.15), 200g of potable water was added, and the pH was adjusted to a pH number between 7 and 8 with the slow addition of potassium carbonate. The mixture was distilled under reduced pressure at a temperature <65°C, and when 210g of solvent was obtained by the distilling, the distilling was stopped. The resultant was extracted with 250g of dichloromethane added, and the water phase was extracted with 250g of dichloromethane for another three times. The dichloromethane phases were combined, and the combined phase was distilled under reduced pressure at a temperature <50°C until no solvent residue. 91.8g of ethyl α-ethyl-2-oxo-1-pyrrolidine acetate was obtained. The total yield of the two-step reaction was 91.8% and the purity was 99.8%.

The quality comparison before and after purification is shown in the table as below.

| sample | purity | Impurity A | Impurity B | Impurity C | Impurity D | Impurity E | Maximum amount of other impurities |
|---|---|---|---|---|---|---|---|
| Before purification | 93.5% | *0.23%* | 0.41% | 2.71% | 0.12% | 2.51% | 0.18% |
| After purification | 99.8% | 0.001% | 0.003% | N.D | N.D | 0.11% | N.D |

### Example 3:

100g of methyl α-ethyl-2-oxo-1-pyrrolidine acetate (purity: 94.7%) prepared in Example 1 was added into a reactor, followed by the addition of 100g of 30wt% potassium hydroxide aqueous solution. The temperature was raised to 35°C, and the reaction was carried out at a maintained temperature of 35°C for 1 hour. After confirming there was no raw material residue by TLC (developing agent: acetone:ethyl acetate=2:3, iodine fumigation color development, the retardation factor value of raw materials: 0.8), the pH of the reaction solution was adjusted to 0.5 with the addition of 36wt% refined hydrochloric acid. When materials were precipitated from the reaction solution along with the addition of hydrochloric acid, the pH adjustment was stopped. The materials were slowly cooled down to 10°C, stirred at a maintained temperature of 10°C for 1 hour, and filtered. The filter cake was dried at 80°C for 6 hours, the moisture content of which was detected to be 0.3wt%, and 85.3g (92.3%) of compound of formula IV was obtained.

The whole batch of compound of formula IV was added into a reactor, followed by the addition of 50g of methanol. The temperature was 0°C, and 10g of 90wt% concentrated sulfuric acid was slowly added dropwise. The temperature was raised to 50°C upon the completion of addition, and the reaction was carried out for 1 hour. After confirming there was no raw material residue by TLC (developing agent: acetone:ethyl acetate=2:3, iodine fumigation color development, the retardation factor value of raw materials: 0.15), 50g of potable water was added, and the pH was adjusted to 6 with the slow addition of potassium bicarbonate. The mixture was distilled under reduced pressure at a temperature ≤70°C, and when 60g of solvent was obtained by the distilling, the distilling was stopped. The resultant was extracted with toluene added, and the water phase was extracted with 100g of toluene for another two times. The toluene phases were combined, and the combined phase was distilled under reduced pressure at a temperature ≤70°C until no solvent residue. 91.5g of methyl α-ethyl-2-oxo-1-pyrrolidine acetate was obtained. The total yield of the two-step reaction was 91.5% and the purity was 99.3%.

### Example 4:

100g of ethyl α-ethyl-2-oxo-1-pyrrolidine acetate (purity: 93.5%) prepared in Example 1 was added into a reactor, followed by the addition of 1000g of 5wt% sodium hydroxide aqueous solution. The temperature was raised to 65°C, and the reaction was carried out at a maintained temperature of 65°C for 5 hours. After confirming there was no raw material residue by TLC (developing agent: acetone:ethyl acetate=2:3, iodine fumigation color development, the retardation factor value of raw materials: 0.8), the pH of the reaction solution was adjusted to 3.0 with the addition of 36wt% refined hydrochloric acid. When materials were precipitated from the reaction solution along with the addition of hydrochloric acid, the pH adjustment was stopped. The materials were slowly cooled down to -10°C, stirred at a maintained temperature of -10°C for 2 hours, and filtered. The filter cake was dried at 100°C for 4 hours, the moisture content of which was detected to be 0.3wt%, and 77.1g(93%) of compound of formula IV was obtained.

The whole batch of compound of formula IV was added into a reactor, followed by the addition of 1000g of ethanol. The temperature was 0°C, and 20g of 98wt% concentrated sulfuric acid was slowly added dropwise. The temperature was raised to 75°C upon the completion of addition, and the reaction was carried out for 5 hours. After confirming there was no raw material residue by TLC (developing agent: acetone:ethyl acetate=2:3, iodine fumigation color development, the retardation factor value of raw materials: 0.15), 500g of potable water was added, and the pH was adjusted to 8 with the slow dropwise addition of sodium carbonate. The mixture was distilled under reduced pressure at a temperature ≤70°C, and when 1000g of solvent was obtained by the distilling, the distilling was stopped. The resultant was extracted with 1000g ethyl acetate added, and the water phase was extracted with 1000g of ethyl acetate for another five times. The ethyl acetate phases were combined, and the combined phase was distilled under reduced pressure at a temperature ≤70°C until no solvent residue. 89.5g of methyl α-ethyl-2-oxo-1-pyrrolidine acetate was obtained. The total yield of the two-step reaction was 89.5% and the purity was 99.8%.

## Claims

1. A method for purifying a levetiracetam intermediate, wherein the method comprises the following steps:
(1) hydrolyzing a crude product of a compound of formula III under alkaline conditions, followed by acidifying, then crystallizing to obtain a compound of formula IV; and
(2) subjecting the compound of formula IV to an esterification reaction, followed by separation, to obtain a purified compound of formula III;
wherein the compound of formula III is 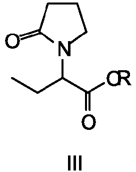 , R is methyl or ethyl, and
the compound of formula IV is

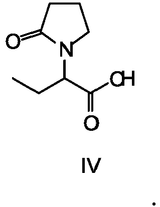

2. The method according to claim 1, wherein the step (1) comprises the following steps:
(a) mixing the crude product of the compound of formula III with an aqueous solution of inorganic alkali, and then heating and performing reaction;
(b) adjusting pH to a pH number between 0.5 and 3.5; and
(c) lowering temperature, and crystallizing, filtering and drying a mixture obtained in step (b) to obtain the compound of formula IV.

3. The method according to claim 2, wherein in step (a),
a temperature of the reaction is 35°C to 65°C, and reaction time is 1 to 5 hours; and
the inorganic alkali is one selected from the group consisting of sodium hydroxide and potassium hydroxide, a concentration of the aqueous solution of inorganic alkali is 5wt% to 30wt%, and a weight of the aqueous solution of inorganic alkali is 1 to 10 times a weight of the compound of formula III.

4. The method according to claim 3, wherein in step (a),
the temperature of the reaction is 40°C to 45°C;
the inorganic alkali is sodium hydroxide.

5. The method according to claim 2, wherein in step (b), the pH is adjusted to a pH number between 2.0 and 2.5.

6. The method according to claim 2, wherein in step (c), a temperature after lowering temperature is -10°C to 10°C, a duration of the crystallization is 1 hour to 2 hours, a temperature for the drying is 80°C to 100°C, and an end point of the drying is until a moisture content ≤0.5wt%.

7. The method according to claim 1, wherein the step (2) comprises the following steps:
(d) adding an alcohol solvent into the compound of formula IV, adding an esterification reaction catalyst, and then heating and performing reaction;
(e) adding water, adjusting the pH to a pH number between 6 and 8 with alkali, and distilling; and
(f) extracting with an organic solvent, and distilling an obtained extract to obtain the purified compound of formula III.

8. The method according to claim 7, wherein in step (d),
the alcohol solvent is selected from the group consisting of methanol, ethanol, and a mixture thereof, and a weight of the alcohol solvent is 0.5 to 10 times a weight of the compound of formula III;
the esterification reaction catalyst is 90wt% to 98wt% concentrated sulfuric acid, and a weight of the concentrated sulfuric acid is 0.01 to 0.2 times the weight of the compound of formula III; and
a temperature of the reaction is 50°C to 75°C and reaction time is 1 hour to 5 hours.

9. The method according to claim 7, wherein in step (e),
a weight of added water is 0.5 to 5 times the weight of the compound of formula III;
the alkali is one or more selected from the group consisting of sodium hydroxide, potassium hydroxide, sodium carbonate, potassium carbonate, sodium bicarbonate and potassium bicarbonate; and
a weight of distilled solvent is 0.8 to 1.2 times a weight of added alcohol solvent, and a temperature of the distilling is ≤70°C.

10. The method according to claim 7, wherein in step (f), the organic solvent is one or more selected from the group consisting of toluene, benzene, dichloromethane and ethyl acetate, the extraction is performed 2 to 5 times, a weight of the organic solvent for a single extraction is 1 to 10 times the weight of the compound of formula III, and a temperature of the distilling is ≤70°C.
